# EUROPEAN PATENT APPLICATION

(11) **EP 3 659 464 A1**
(43) Date of publication of application: **03.06.2020**
(21) Application number: 19170146.5
(22) Date of filing: 18.04.2019
(51) Int. Cl.: A45D 29/04, A61B 17/54, B24D 3/34

(54) **BEAUTY TOOL FOR NAIL AND SKIN EXFOLIATION AND METHOD OF PRODUCING THE SAME**

(30) Priority: 30.11.2018 KR 20180152729; 02.04.2019 KR 20190038276
(71) Applicant: Dains Co., Ltd., Jeonju-si, Jeollabuk-ro (KR)
(72) Inventor: PARK, Chun Seong, Suwon-si, Gyeonggi-do (KR); KIM, Sung Jung, Suwon-si, Gyeonggi-do (KR)
(74) Representative: Weickmann & Weickmann PartmbB

(57) **Abstract**

The present disclosure relates to a beauty tool for nail and skin exfoliation, and specifically, a beauty tool for nail and skin exfoliation including a body, a plate which is coupled to the body and is made of a glass material, a plurality of metal protrusions which are disposed on an upper surface of the plate with a predetermined pattern, and a plurality of concave grooves which are defined with a predetermined depth on the upper surface of the plate and are not covered by the plurality of metal protrusions.

## Description

### CROSS-REFERENCE TO RELATED APPLICATION

This application claims priority to and the benefit of Korean Patent Application No. 10-2019-0038276 filed in the Korean Intellectual Property Office on April 02, 2019, the entire contents of which are incorporated herein by reference.

### BACKGROUND OF THE INVENTION

### 1. Field of the Invention

The present invention relates to a beauty tool for nail and skin exfoliation and a method of producing the beauty tool, particularly, a beauty tool for nail and skin exfoliation and a method of producing the beauty tool which prevents a the skin from being irritated and damaged, can be used by everyone, and is applicable to various parts of the body.

### 2. Description of the Related Art

In general, a horny layer is formed at the skin's epidermis. The horny layer is the outermost layer of the skin and has an effect of protecting the skin from external stimulus.

However, in an area of the body, which is under pressure due to a frequent contact with the external stimulus, such as the heel, the horny layer is thickened and hardened. If the horny layer is left untouched, dead skin cells are cracked and the soft skin inside the dead skin cells is cracked, which causes pain, aging, and bad appearance.

Therefore, in order to remove the horny layer, the dead skin cells are soaked in hot water to be scraped off or removed by using a knife but, while removing the dead skin cells, the skin is damaged, safety thereof is deteriorated and it is unsanitary.

In order to solve such a problem, various types of beauty tools for nail and skin exfoliation have been developed. For example, a beauty tool for nail and skin exfoliation produced by processing an irregular stone of basalt type has been developed, but a surface pattern thereof is irregular, resulting in deterioration of dead skin cell removal efficiency, and the skin is damaged due to a high surface roughness.

Therefore, in order to solve this problem, Callus Removing Plate by Micro-cutters and Callus Remover disclosed in Korean Patent No. 10-1184144 have been developed, but there is a problem that, since polishing performance is deteriorated and the callus remover is repeatedly used until being discarded, cleaning and disinfection are essential, and an abrasive portion of a surface is worn by a cleaning tool and disinfectant, which causes the life of the remover to be reduced.

### SUMMARY OF THE INVENTION

An exemplary embodiment of the present invention provides a beauty tool for nail and skin exfoliation and a method of producing the beauty tool which prevents a skin from being irritated and damaged, can be used by everyone, and is applicable to various parts of the body.

According to one embodiment of the present discloser, a beauty tool for nail and skin exfoliation is provided. The beauty tool includes a body, a plate which is coupled to the body and is made of a glass material, a plurality of metal protrusions which are disposed on an upper surface of the plate with a predetermined pattern, and a plurality of concave grooves which are defined with a predetermined depth on the upper surface of the plate and are not covered by the plurality of metal protrusions.

The plate may have a thickness in a range of 0.1 mm to 5 mm.

Each of the plurality of metal protrusions may have a thickness in a range of 10 nm to 1000 nm.

Each of the plurality of metal protrusions may be made of one or more materials selected from gold, molybdenum, tungsten, and chromium.

Each of the plurality of metal protrusions may have a circular shape and is disposed on the plate which has an area extended from a center on a plane, in which a plane diameter of the each of the plurality of metal protrusions is reduced as disposed away from the center.

Each of the plurality of concave grooves may have a tapered shape with a rounded end.

Each of the plurality of concave grooves may have a depth in a range of 10 µm to 100 µm.

The beauty tool may further include a design portion which is disposed on one side of the body and shows various designs.

The beauty tool may further include a sand portion which includes protrusions disposed on a surface thereof in an irregular pattern and which is detachably attached to the body.

The beauty tool may further include a coating layer which covers the plate, sides of the plurality of metal protrusions, and the plurality of concave grooves except an upper surface of the plurality of metal protrusions.

The beauty tool may further include a microprotrusion interposed between the plate and the plurality of metal protrusions, wherein an upper surface of the plurality of metal protrusions corresponds to a shape of the microprotrusion.

According to another embodiment of the present disclosure, a method of producing a beauty tool for nail and skin exfoliation is provided. The method includes preparing a body, preparing a plate made of glass, attaching a first shadow mask to a surface of the plate, depositing a metal film on the surface of the plate to where the first shadow mask does not cover, removing the first shadow mask, forming a plurality of concave grooves by etching an upper surface of the plate on which the metal film is not deposited, and coupling the plate to the body.

The metal film may be deposited through a sputtering process.

The method may further include forming a design portion which is disposed on one side of the body and shows various designs.

The method may further include forming a sand portion in which a plurality of protrusions are formed on a surface of the sand portion in an irregular pattern through a sandblasting process, wherein the sand portion is coupled to the body.

The method may further include attaching a plurality of second shadow masks to an upper surface of the metal film after the step of the forming the plurality of concave grooves, a coating liquid application step of applying a coating liquid to an upper side of the plate to which the second shadow masks does not cover, and removing the second shadow masks.

The method may further include attaching a plurality of third shadow masks to the upper surface of the plate after the step of the preparing the body, etching the upper surface of the plate to which the third shadow masks do cover to form microprotrusions, and removing the third shadow masks.

According to a beauty tool for nail and skin exfoliation and a method of producing the beauty tool of the present invention configured as described above, a side surface of a concave groove is in contact with an upper surface of a plate in an obtuse angle of a rounded shape, and thus, there is an effect that a skin is prevented from being irritated and damaged.

In addition, since sterilization and disinfection occur at a high temperature, higher than or equal to 150°C may be performed, this has an effect that prevents the bacteria from propagating.

In addition, dead skin cells are not accumulated at an internal corner of the groove, and thus, it is possible for the beauty tool for nail and skin exfoliation to be easily washed with running water.

In addition, since the beauty tool for nail and skin exfoliation may be manufactured without a separate photolithography step and a metal film etching step, the production cost is reduced and the defective rate is lowered.

In addition, it is possible to provide the beauty tool for nail and skin exfoliation which can be used by everyone and is usable for various parts of the body such as hands and feet.

### BRIEF DESCRIPTION OF THE DRAWINGS

FIGS. 1A and 1B are perspective views of a beauty tool for nail and skin exfoliation according to a first exemplary embodiment of the present invention.
FIG. 2 is an exemplary diagram of a plate according to the first exemplary embodiment of the present invention.
FIGS. 3A to 3C are cross-sectional views taken along a line A-A' of the beauty tool for nail and skin exfoliation according to the first exemplary embodiment of the present invention.
FIG. 4 is an exemplary diagram illustrating a sand portion of the beauty tool for nail and skin exfoliation according to the first exemplary embodiment of the present invention.
FIG. 5 is a cross-sectional diagram illustrating a beauty tool for nail and skin exfoliation according to a second exemplary embodiment of the present invention.
FIG. 6 is a cross-sectional diagram illustrating a beauty tool for nail and skin exfoliation according to a third exemplary embodiment of the present invention.
FIG. 7 is a flowchart illustrating a method of producing the beauty tool for nail and skin exfoliation according to the first exemplary embodiment of the present invention.
FIG. 8 is a flowchart illustrating a method of producing the beauty tool for nail and skin exfoliation according to the second exemplary embodiment of the present invention.
FIG. 9 is a flowchart illustrating a method of producing the beauty tool for nail and skin exfoliation according to the third exemplary embodiment of the present invention.

### DETAILED DESCRIPTION OF THE EMBODIMENTS

Hereinafter, description on the present invention with reference to the drawings is not limited to a specific exemplary embodiment, various changes may be applied thereto, and various exemplary embodiments may be made. It is to be understood that the following description covers all changes, equivalents, and alternatives falling within the spirit and scope of the present invention.

In the following description, the terms first, second, and the like are used to describe various configuration elements, are not limited to their own meaning, and are used only for the purpose of distinguishing one configuration element from another configuration element.

Like reference numerals used throughout the specification denote like elements.

The singular forms used in the present invention include plural forms unless the context clearly dictates otherwise. It should be construed that the term "include", "comprise", "have", or the like is intended to designate the presence of features, a numeral, a step, an operation, a configuration element, a component, or a combination thereof described in the specification, and it is to be understood that the presence or addition of one or more other features, a numeral, a step, an operation, a configuration element, a component, or a combination thereof is not precluded.

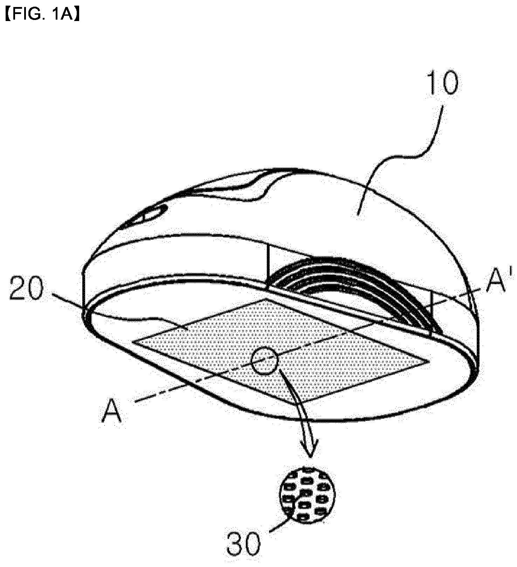

A beauty tool for nail and skin exfoliation and a method of producing the beauty tool according to an exemplary embodiment of the present invention will now be described in detail with reference to FIGS. 1A to 9.

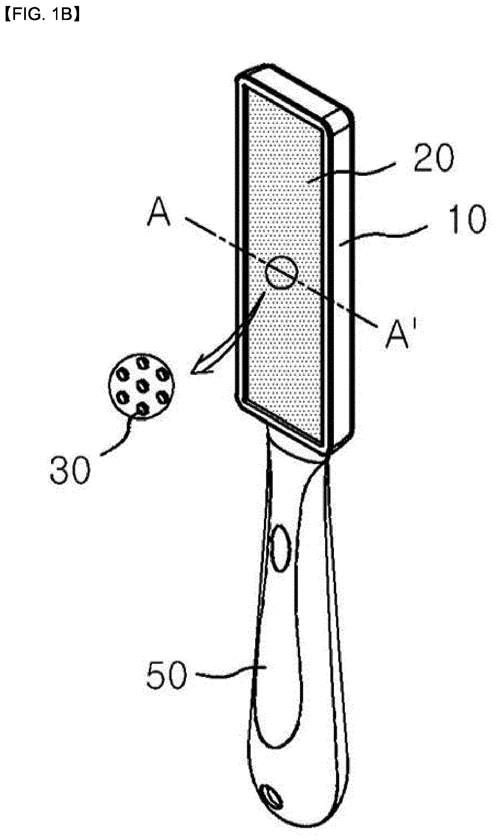

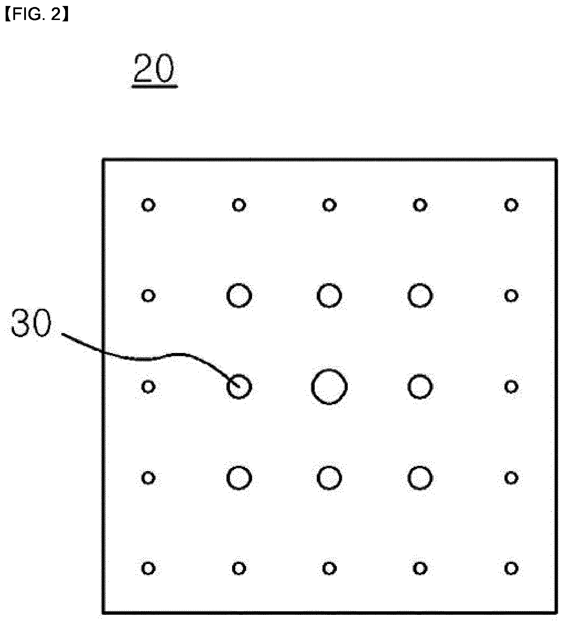

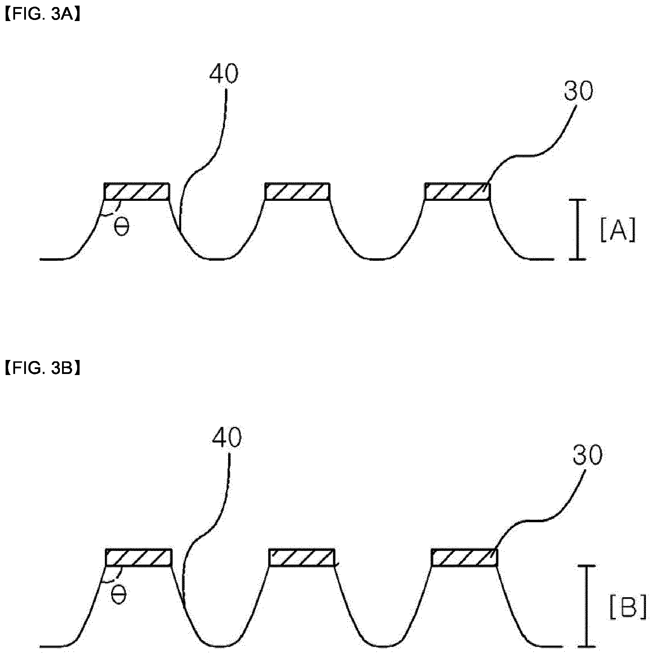

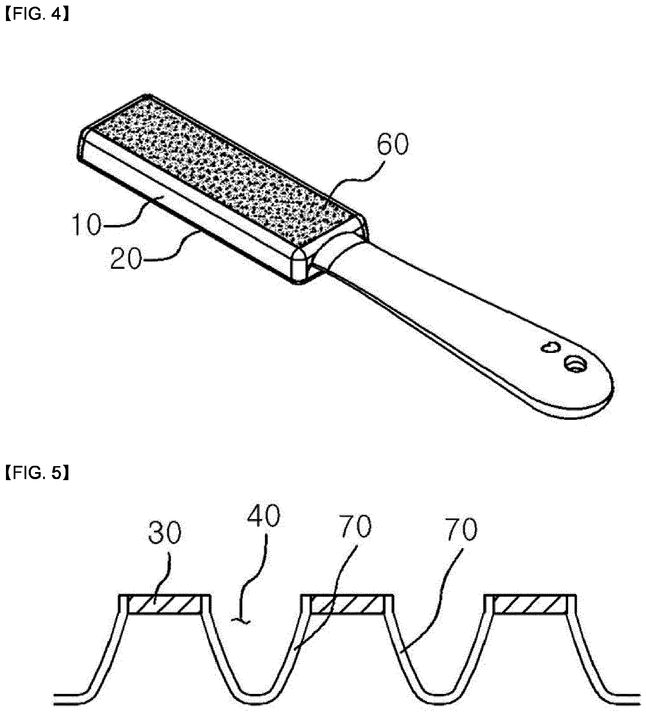

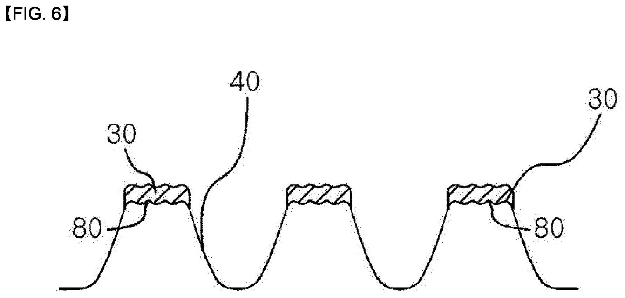

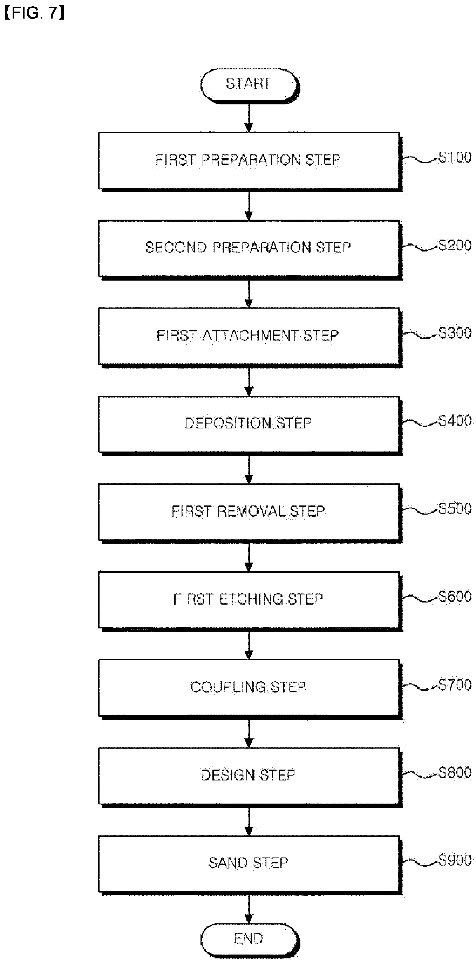

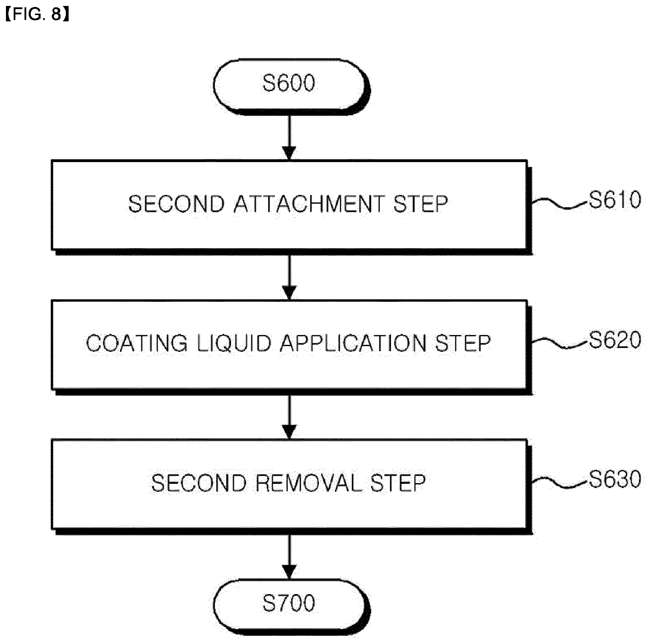

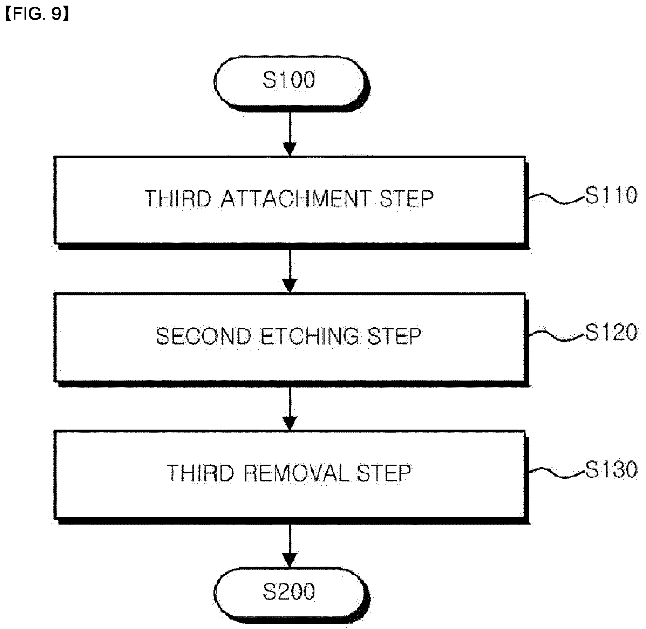

FIGS. 1A and 1B are perspective views of a beauty tool for nail and skin exfoliation according to a first exemplary embodiment of the present invention, FIG. 2 is an exemplary diagram of a plate according to the first exemplary embodiment of the present invention, FIGS. 3A to 3C are cross-sectional views taken along a line A-A' of the beauty tool for nail and skin exfoliation according to the first exemplary embodiment of the present invention, FIG. 4 is an exemplary diagram illustrating a sand portion of the beauty tool for nail and skin exfoliation according to the first exemplary embodiment of the present invention, FIG. 5 is a cross-sectional diagram illustrating a beauty tool for nail and skin exfoliation according to a second exemplary embodiment of the present invention, FIG. 6 is a cross-sectional diagram illustrating a beauty tool for nail and skin exfoliation according to a third exemplary embodiment of the present invention, FIG. 7 is a flowchart illustrating a method of producing the beauty tool for nail and skin exfoliation according to the first exemplary embodiment of the present invention, FIG. 8 is a flowchart illustrating a method of producing the beauty tool for nail and skin exfoliation according to the second exemplary embodiment of the present invention, and FIG. 9 is a flowchart illustrating a method of producing the beauty tool for nail and skin exfoliation according to the third exemplary embodiment of the present invention.

Recently, an exfoliation device in which fine protrusions are formed on a glass plate using a photolithography process is being developed. The beauty tool for nail and skin exfoliation of the related art which is produced through the photolithography process is mostly formed in a curved shape having a smaller diameter in the middle portion than an upper portion of the protrusion, and thereby, the projection and the surface of the glass plate are formed in a shape in contact with an acute angle.

Although dead skin cell removal increases irritation and damage caused to fragile skin having thin dead skin cells; a washing brush or the like is used to remove the dead skin cells which are interposed between pattern grooves at the time of washing, and thereby, a problem that the pattern groove is damaged occurs.

Therefore, the present invention is developed in view of the need for a beauty tool for nail and skin exfoliation which prevents the skin from being irritated and damaged because the side surface of a concave groove and an upper surface of the plate are in contact with each other in an obtuse angle, prevents dead skin cells from accumulating at an internal corner of the groove, may be easily washed with running water, and is usable for everyone.

As illustrated in FIGS. 1A to 4, a beauty tool for nail and skin exfoliation according to a first exemplary embodiment of the present invention may include a body 10, a plate 20, a metal protrusion 30, and a concave groove 40.

First, since the body 10 is formed of a glass material, a metal material, or a plastic material thereby being able to be sterilized and disinfected at a high temperature, bacteria may be prevented from propagating.

Specifically, when the body 10 is formed of the glass material, the body may be sterilized and disinfected at a temperature lower than or equal to 500°C, when the body 10 is formed of the metal material, the body may be sterilized and disinfected at a temperature lower than or equal to 300°C, and when the body 10 is formed of the plastic (ABS or the like) material, the body may be sterilized and disinfected at a temperature lower than or equal to 150°C.

At this time, the body 10 may be formed into a mouse shape so as to have an excellent grip feeling.

However, since this is only an exemplary embodiment of the present invention, the body 10 may be formed into a bar or an oval shape for easy use by a user as another exemplary embodiment of the present invention.

Next, the plate 20 is detachably coupled to the body 10, and thus, the plate 20 may be disinfected and cleaned by separating the plate 20 from the body 10.

At this time, the plate 20 may be coupled to one surface of the body 10, which is merely an exemplary embodiment of the present invention, and thus, a pair of plates 20 may be coupled to one surface and the other surface of the body 10 or may be integrally coupled to one surface of the body 10.

In addition, the plate 20 may be formed of a special glass material having a high durability, and thus, the plate may be sterilized and disinfected at a temperature lower than or equal to 500°C, thereby, preventing bacteria from propagating.

However, this is only an exemplary embodiment of the present invention, and the plate may be formed of a film material such as polyester (PET).

In addition, the plate 20 may be formed to have a thickness of 0.1 mm to 5 mm.

At this time, if the thickness is less than 0.1 mm, there is a risk of breakage due to weakness to impact, and if the thickness exceeds 5 mm, not only there is a sense of difference when joining to the body 10 but also a grip feeling may be deteriorated.

Next, the metal protrusion 30 may be formed in a shape in which the metal protrusion is stacked on an upper surface of the plate 20.

In addition, the metal protrusion 30 may be formed of at least one of the following metal materials, gold, molybdenum, tungsten, or chromium.

Specifically, the metal protrusion 30 may be formed by attaching a first shadow mask on the upper surface of the plate 20, and then, by depositing a metal on the upper surface of the plate 20 to which the first shadow mask is not attached through a sputtering process.

At this time, it is preferable that the plurality of holes are formed in the first shadow mask, and the first shadow mask is attached to the upper surface of the plate 20 by using one of a double-sided tape and a surface magnet and is removed from the upper surface of the plate 20 after the metal protrusion 30 is formed.

Therefore, the upper surface of the plate 20 is protected, and the surface strength of the beauty tool is increased so as to be able to have a high durability.

In addition, the metal protrusion 30 may be formed to a thickness of 10 nm to 1000 nm.

At this time, if the thickness is less than 10 nm, the surface strength is not sufficiently increased, and if the thickness exceeds 1000 nm, a production cost may increase.

In addition, the metal protrusions 30 may be arranged in a constant pattern.

For example, the metal protrusion 30 may be formed in an equilateral triangle such that adjacent metal protrusions 30 have the same interval. So, dead skin cells may be removed.

As another example, the metal protrusion 30 may be formed in a square such that adjacent metal protrusions 30 have the same interval.

However, since this is only an exemplary embodiment of the present invention, the metal protrusions 30 may be arranged in various patterns.

In addition, as illustrated in FIG. 2, the metal protrusions 30 may be formed such that a plane diameter decreases toward an outer side with respect to an inner side of the plate 20.

Specifically, diameters of the plurality of holes formed in the first shadow mask may be reduced toward the outer side with respect to the inner side.

Therefore, a delicate exfoliation may be performed toward the outer side of a curved nail and an outer side of a heel may be easily trimmed.

The concave groove 40 may be formed at a position where the metal protrusion 30 is not formed and may be formed as a groove etched to a predetermined depth from the upper surface of the plate 20.

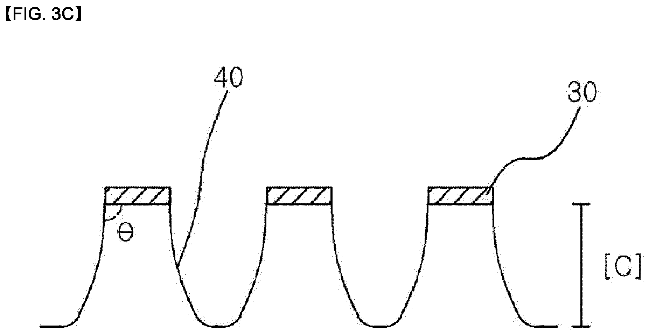

In addition, as illustrated in FIG. 3 the concave grooves 40 may be formed to be connected to the metal protrusions 30 in a rounded shape. More specifically, the metal protrusion 30 may be in contact with the upper side of the concave groove 40 in the form of an obtuse angle θ.

Accordingly, since dead skin cells are not accumulated in a joint between the metal protrusion 30 and the concave groove 40, the beauty tool for nail and skin exfoliation may be hygienically managed even by simple water washing.

Since above description is only an exemplary embodiment of the present invention, a normal cross section of the concave groove 40 may be formed in a trapezoidal shape.

In addition, the concave groove 40 is formed to a depth of 5 to 100 µm, and at this time, various users may use the beauty tool for nail and skin exfoliation according to the present invention depending on the depth of the concave groove 40.

At this time, if the depth of the concave groove 40 is less than 5 µm, a dead skin cell removal effect is reduced, and if the depth is greater than 100 µm, irritation occurs excessively when the beauty tool for nail and skin exfoliation is used, and thereby,the user's skin may be damaged.

For example, in order to provide a beauty tool for nail and skin exfoliation suitable for generating gloss on the nail, the concave groove 40 may be formed to have a depth A of 10 to 35 µm as illustrated in FIG. 3A.

As another example, in order to provide a beauty tool for nail and skin exfoliation suitable for skin exfoliation, the concave groove 40 may be formed to have a depth B of 10 to 50 µm as illustrated in FIG. 3B.

At this time, as the depth of the concave groove 40 becomes deeper, not only the degree of grinding may be improved, but also accumulated thick dead skin cells may be easily removed.

As still another example, in order to provide the beauty tool for nail and skin exfoliation suitable for foot exfoliation, the concave groove 40 may be formed to a depth C of 50 to 100 µm as illustrated in FIG. 3C.

According to the present invention, it is possible to provide a beauty tool for nail and skin exfoliation which may be used for various parts of the body of a user.

The beauty tool for nail and skin exfoliation according to the present invention may further include a design portion 50.

The design portion 50 may be formed on one side of the body 10 and various coatings and designs may be applied to the design portion 50.

As an example, a mirror coating on the surface makes it easier to see an elbow, a heel, and the like which are hard to directly see.

This is only an exemplary embodiment of the present invention, and various designs such as a product name, a company logo, an initial, and a design pattern may be shown.

In addition, the design portion 50 may be formed of an epoxy material, and thus, disinfection may be performed at a temperature lower than or equal to 150°C, and thereby, bacteria may be prevented from propagating.

As another example, the design portion 50 may be formed of a silicone material to prevent scattering when the body 10 is damaged and to absorb an external impact.

This is only an exemplary embodiment of the present invention, and the design portion may be formed of various films and materials that may prevent the scattering.

In addition, as illustrated in FIG. 4, the beauty tool for nail and skin exfoliation according to the exemplary embodiment of the present invention may further include a sand portion 60.

The sand portion 60 may be detachably coupled to the other surface of the body 10, and thus, the sand portion 60 may be separated from the body 10 to be sterilized and cleaned.

In addition, when a surface of the sand portion 60 is polished due to use for a long time and thereby an exfoliation function thereof is deteriorated, the sand portion may be easily replaced.

The sand portion is only an exemplary embodiment of the present invention and may be integrally coupled to the other surface of the body 10.

In addition, the sand portion 60 may include protrusions formed on the surface in an irregular pattern, and more specifically, a plurality of the protrusions may be formed on the surface through a sandblasting process.

As described above, according to the beauty tool for nail and skin exfoliation of the present invention, since a side surface of the concave groove is in contact with an upper surface of the plate in a rounded obtuse angle shape, skin is prevented from being irritated and damaged.

In addition, since no dead skin cell is accumulated in a joint between the metal protrusion 30 and the concave groove 40, the beauty tool for nail and skin exfoliation may be hygienically managed even by simple water washing.

In addition, it is possible to provide the beauty tool for nail and skin exfoliation which may be easily used by everyone and may safely remove dead skin cells from various body parts such as hands and feet by adjusting a depth of a concave groove.

Meanwhile, another example, a coating layer 70 may be formed on an upper surface of the plate 20 except an upper surface of the metal protrusion 30 such that the removed dead skin cells may be easily separated.

Hereinafter, a beauty tool for nail and skin exfoliation according to a second exemplary embodiment of the present invention will be described with reference to FIG. 5.

Referring to FIG. 5, the beauty tool for nail and skin exfoliation according to the second exemplary embodiment of the present invention may include the coating layer 70 formed on the upper surface of the plate 20 except the upper surface of the metal protrusion 30 and may be configured in substantially the same manner as in the first exemplary embodiment except the coating layer 70.

Therefore, description on the duplicate configurations will be omitted, and only differences therebetween will be described below.

The coating layer 70 may be formed on the upper surface of the plate 20 except the upper surface of the metal protrusion 30.

At this time, the coating layer 70 may be formed of a material excellent in waterproof property, such as aqueous urethane or polyurea, to easily remove the dead skin cells which are removed from the body of a user and deposited on the concave groove 40 and to easily dry the beauty tool for nail and skin exfoliation at the time of cleaning with water.

Specifically, the coating layer 70 may be formed by attaching a plurality of second shadow masks to the upper surface of the metal protrusion 30, and then, by depositing a coating liquid on the upper surface of the plate 20 to which the second shadow masks are not attached through a sputtering process.

The coating layer 70 may be preferably formed on a side surface of the metal protrusion 30 and the plate 20 except the upper surface of the metal protrusion 30.

Accordingly, the metal protrusions 30 stacked on the upper surface of the plate 20 may be prevented from being peeled off from the plate 20, and thus, the durability of the beauty tool for nail and skin exfoliation increases.

At this time, it is preferable that the second shadow mask is formed to have the same size as the upper surface of the metal protrusion 30, is attached to the upper surface of the metal protrusion 30 by one of a double-sided tape and a surface magnet, and is removed from the upper surface of the metal projection 30 after the coating layer 70 is formed.

As another example, a micro-protrusion 80 may be formed on the upper side of the plate 20 so as to increase a frictional force with the hand, foot, and skin.

Hereinafter, a beauty tool for nail and skin exfoliation according to a third exemplary embodiment of the present invention will be described with reference to FIG. 6.

Referring to FIG. 6, the beauty tool for nail and skin exfoliation according to the third exemplary embodiment of the present invention may include a configuration of micro-protrusion 80 formed on the upper side of the plate 20 and may be configured substantially with the same as in the first exemplary embodiment except the micro-protrusion 80.

Therefore, description on the duplicate configurations will be omitted, and only differences will be described below.

The micro-protrusion 80 may be formed on the upper side of the plate 20.

More specifically, the micro-protrusion 80 may be formed by attaching a plurality of third shadow masks to the upper surface of the plate 20 and then, by etching the upper surface of the plate 20 to which the third shadow mask is not attached.

At this time, the metal protrusions 30 may be stacked on an upper side of the micro-protrusion 80, and the upper surface of the stacked metal protrusion 30 may correspond to the micro-protrusion 80.

Accordingly, a surface area of the metal protrusion 30 increases, and thereby, a frictional force with the hand, foot, and skin increases.

In addition, a contact area between the micro-protrusion 80 and the metal protrusion 30 increases, and thereby, the metal protrusion 30 may be prevented from being peeled off from the micro-protrusion 80.

At this time, it is preferable that the third shadow mask is formed in a minute size, is attached to the upper surface of the plate 20 by one of a double-faced tape,or a surface magnet, and is removed from the upper surface of the plate 20 after the micro-protrusion 80 is formed.

The methods of producing the beauty tool for nail and skin exfoliation according to exemplary embodiments of the present invention will be described below.

As illustrated in FIG. 7, a method of producing the beauty tool for nail and skin exfoliation according to a first exemplary embodiment of the present invention includes a first preparation step S100, a second preparation step S200, a first attachment step S300, a deposition step S400, a first removal step S500, a first etching step S600, a coupling step S700, a design step S800, and a sand step S900.

First, the first preparation step S100 is a step of preparing the body 10 and may be configured by a step of preparing a mouse, a bar, and the elliptical body 10 for easy use by a user with an excellent grip feeling.

Next, the second preparation step S200 is a step of preparing the plate 20, wherein the plate 20 may be formed of a special glass material having a high durability .

Next, the first attachment step S300 may be configured by a step of attaching a first shadow mask to a surface of the plate (20).

At this time, the first shadow mask may include a plurality of holes and may be attached by using one of a double-sided tape and a surface magnet.

Accordingly, the first shadow mask may be easily removed from the surface of the plate 20 thereafter.

In addition, the first shadow mask may be formed in a shape in which a diameter of the hole is reduced toward the outer side with respect to an inner side of the plate 20.

Next, the deposition step S400 may be configured by a step of depositing a metal film on the surface of the plate 20 to which the first shadow mask is not attached.

Specifically, the metal film may be deposited on the surface of the plate 20 by depositing the metal film on the holes of the first shadow mask.

At this time, the metal film may be deposited through a sputtering process, and thereby, the metal protrusion 30 may be formed on the surface of the plate 20.

In addition, the metal film may be formed in a shape in which a plane diameter is reduced toward the outer side with respect to the inner side along the diameter of the hole of the first shadow mask.

Next, the first removal step S500 may be configured by a step of removing the first shadow mask.

Next, the first etching step S600 may be configured by a step of forming the concave groove 40 by etching the upper surface of the plate 20 on which the metal film is not deposited by using a spraying or dipping method.

Next, the coupling step S700 is a step of coupling the plate 20 on which the concave groove 40 is formed to the body 10, and at this time, the plate 20 may be formed in a shape in which one plate is coupled to one surface of the body 10.

This is only an exemplary embodiment of the present invention, and a pair of plates 20 may be coupled to one surface and the other surface of the body 10, respectively.

Next, the design step S800 is a step of forming the design portion 50 showing various designs, and at this time, the design portion 50 may be formed on one side of the body 10.

Next, the sand step S900 is a step of forming the sand portion 60 and may be configured by a step of forming the sand portion 60 in which a plurality of protrusions of an irregular pattern are formed on the surface through a sandblasting process.

At this time, one sand portion 60 may be coupled to the other surface of the body 10.

According to the above-described production method, since a metal film is deposited on an upper surface of the plate 20 using a shadow mask, it is possible to manufacture a beauty tool for nail and skin exfoliation without using a separate photolithography step and a metal film etching step.

Accordingly, since the method of producing the beauty tool for nail and skin exfoliation is simplified, not only are production costs reduced but also the defective rate is lowered.

As illustrated in FIG. 8, a method of producing a beauty tool for nail and skin exfoliation according to a second exemplary embodiment of the present invention may further include a second attachment step S610, a coating liquid application step S620, and a second removal step S630 subsequent to the first etching step S600.

Hereinafter, the method of producing the beauty tool for nail and skin exfoliation according to the second exemplary embodiment of the present invention will be described with reference to FIG. 8.

Referring to FIG. 8, the method of producing the beauty tool for nail and skin exfoliation according to the second exemplary embodiment of the present invention may further include the second attachment step S610, the coating liquid application step S620, and the second removal step S630, and may be configured in substantially the same manner as in the first exemplary embodiment except the three steps.

Therefore, description on the duplicate configurations will be omitted, and only differences will be described below.

The second attachment step S610 may be configured by a step of attaching a plurality of second shadow masks to the upper surface of the metal film.

At this time, the second shadow mask may be formed to have the same size as the upper surface of the metal protrusion 30 and may be attached by using one of a double-sided tape and a surface magnet.

Accordingly, the second shadow mask may be easily removed from the upper surface of the metal projection 30 thereafter.

The coating liquid application step S620 may be configured by a step of forming the coating layer 70 by applying a coating liquid to the upper surface of the concave groove 40 to which the second shadow mask is not attached.

At this time, the coating liquid may be formed of a material excellent in waterproof property, such as aqueous urethane or polyurea to easily remove the dead skin cells which are removed from the body of a user and deposited on the concave groove 40 and to easily dry the beauty tool for nail and skin exfoliation at the time of cleaning with water.

The second removal step S630 may be configured by a step of removing the second shadow mask.

In addition, as illustrated in FIG. 9, a method of producing a beauty tool for nail and skin exfoliation according to a third exemplary embodiment of the present invention may include a third attachment step S110, a second etching step S120, and a third removal step S130 subsequent to the third preparation step S100.

Hereinafter, the method of producing the beauty tool for nail and skin exfoliation according to the third exemplary embodiment of the present invention will be described with reference to FIG. 9.

Referring to FIG. 9, the method of producing the beauty tool for nail and skin exfoliation according to the third exemplary embodiment of the present invention may further include the third attachment step S110, the second etching step S120, and the third removal step S130, and may be configured in substantially the same manner as in the first exemplary embodiment except the three steps.

Therefore, description on the duplicate configurations will be omitted, and only differences will be described below.

The third attachment step S110 may be configured by a step of attaching a plurality of third shadow masks to the upper surface of the plate 20.

At this time, the third shadow mask is formed in a minute size and may be attached by using one of a double-sided tape and a surface magnet.

Accordingly, the third shadow mask may be easily removed from the upper surface of the plate 20 thereafter.

The second etching step S120 may be configured by a step of forming the micro-protrusion 80 by etching the upper surface of the plate 20 to which the third shadow mask is not attached.

The third removal step S130 may be configured by a step removing the third shadow mask.

Since the metal protrusion 30 is stacked on an upper side of the micro-protrusion 80 and thereby the upper surface of the metal protrusion 30 corresponds to the micro-protrusion 80, a frictional force with the skin increases.

While first, second, and third exemplary embodiments of the present invention are described above with reference to the accompanying drawing, and it is to be understood that the exemplary embodiments may be embodied in many other specific forms by those skilled in the art to which the present invention belongs. Therefore, the exemplary embodiments described above are illustrative in all respects and are not restrictive.

Although the first, second, and third exemplary embodiments are described for the purpose of specifically describing the present invention, the exemplary embodiments are to describe the present invention more clearly, and it is apparent that the first, second, and third exemplary embodiments may be applied to one another.
The present disclosure relates to a beauty tool for nail and skin exfoliation, and specifically, a beauty tool for nail and skin exfoliation including a body, a plate which is coupled to the body and is made of a glass material, a plurality of metal protrusions which are disposed on an upper surface of the plate with a predetermined pattern, and a plurality of concave grooves which are defined with a predetermined depth on the upper surface of the plate and are not covered by the plurality of metal protrusions.

### <Description of symbols>

- 10:: body
- 20:: plate
- 30:: metal protrusion
- 40:: concave groove
- 50:: design portion
- 60:: sand portion
- 70:: coating layer
- 80:: microprotrusion

## Claims

1. A beauty tool for nail and skin exfoliation comprising:
a body (10);
a plate (20) which is coupled to the body (10) and is made of a glass material;
a plurality of metal protrusions (30) which are disposed on an upper surface of the plate (20) with a predetermined pattern; and
a plurality of concave grooves (40) which are defined with a predetermined depth on the upper surface of the plate (20) and are not covered by the plurality of metal protrusions (30).

2. The beauty tool for nail and skin exfoliation of claim 1, wherein the plate (20) has a thickness in a range of 0.1 mm to 5 mm.

3. The beauty tool for nail and skin exfoliation of claim 1 or 2, wherein each of the plurality of metal protrusions (30) has a thickness in a range of 10 nm to 1000 nm, and/or
wherein each of the plurality of metal protrusions (30) is made of one or more materials selected from gold, molybdenum, tungsten, and chromium.

4. The beauty tool for nail and skin exfoliation of any of claims 1 to 3, wherein each of the plurality of metal protrusions (30) has a circular shape and is disposed on the plate (20) which has an area extended from a center on a plane, in which a plane diameter of the each of the plurality of metal protrusions (30) is reduced as disposed away from the center.

5. The beauty tool for nail and skin exfoliation of any of claims 1 to 4, wherein each of the plurality of concave grooves (40) has a tapered shape with a rounded end, and/or
wherein each of the plurality of concave grooves (40) has a depth in a range of 10 µm to 100 µm.

6. The beauty tool for nail and skin exfoliation of any of claims 1 to 5, further comprising:
a design portion (50) which is disposed on one side of the body (10) and shows various designs.

7. The beauty tool for nail and skin exfoliation of any of claims claim 1 to 6, further comprising:
a sand portion (60) which includes protrusions disposed on a surface thereof in an irregular pattern and which is detachably attached to the body (10).

8. The beauty tool for nail and skin exfoliation of any of claims 1 to 7, further comprising:
a coating layer (70) which covers the plate (20), sides of the plurality of metal protrusions (30), and the plurality of concave grooves (40) except an upper surface of the plurality of metal protrusions (30).

9. The beauty tool for nail and skin exfoliation of any of claims 1 to 8, further comprising:
a microprotrusion (80) interposed between the plate (10) and the plurality of metal protrusions (30),
wherein an upper surface of the plurality of metal protrusions (30) corresponds to a shape of the microprotrusion (80).

10. A method of producing a beauty tool for nail and skin exfoliation comprising:
preparing a body (10);
preparing a plate (20) made of glass;
attaching a first shadow mask to a surface of the plate (20);
depositing a metal film on the surface of the plate (20) to where the first shadow mask does not cover;
removing the first shadow mask;
forming a plurality of concave grooves (40) by etching an upper surface of the plate (20) on which the metal film is not deposited; and
coupling the plate (20) to the body (10).

11. The method of producing the beauty tool for nail and skin exfoliation of claim 10, wherein the metal film is deposited through a sputtering process.

12. The method of producing the beauty tool for nail and skin exfoliation of claim 10 or 11, further comprising:
forming a design portion (50) which is disposed on one side of the body (10) and shows various designs.

13. The method of producing the beauty tool for nail and skin exfoliation of any of claims 10 to 12, further comprising:
forming a sand portion (60) in which a plurality of protrusions are formed on a surface of the sand portion (60) in an irregular pattern through a sandblasting process,
wherein the sand portion (60) is coupled to the body (10).

14. The method of producing the beauty tool for nail and skin exfoliation of any of claims 10 to 13, further comprising:
attaching a plurality of second shadow masks to an upper surface of the metal film after the step of the forming the plurality of concave grooves (40);
a coating liquid application step of applying a coating liquid to an upper side of the plate (20) to which the second shadow masks does not cover; and
removing the second shadow masks.

15. The method of producing the beauty tool for nail and skin exfoliation of any of claims 10 to 14, further comprising:
attaching a plurality of third shadow masks to the upper surface of the plate (20) after the step of the preparing the body (10);
etching the upper surface of the plate (20) to which the third shadow masks do cover to form microprotrusions (80); and
removing the third shadow masks.
